# EUROPEAN PATENT APPLICATION

(11) **EP 2 044 971 A1**
(43) Date of publication of application: **08.04.2009**
(21) Application number: 08253149.2
(22) Date of filing: 26.09.2008
(51) Int. Cl.: A61M 25/10, A61M 25/00

(54) **A balloon catheter**

(30) Priority: 05.10.2007 JP 2007261445
(71) Applicant: ASAHI INTECC CO., LTD., Nagoya-shi, Aichi-ken (JP)
(72) Inventor: Iwamuro, Shigenobu, Nagoya-shi Aichi-ken (JP); Katsurada, Takeharu, Nagoya-shi Aichi-ken (JP); Kubo, Kazuya, Nagoya-shi Aichi-ken (JP)
(74) Representative: Nicholls, Michael John

(57) **Abstract**

In a balloon catheter (1), a distal end portion (31) of a hypotube (3) has a gradient opening (33), an open surface end of which inclines to angle down distally against the axial direction of the the distal end portion (31), and having a planar opening (34). A distal end portion (41) of a core wire (4) is inserted into a distal end side of a shaft (2). A rear end portion (42) of the core wire (4) is fixedly inserted into the distal end portion (31) of the hypotube (3), and having a cross sectional area smaller than a cross sectional area of a boundary (43) between the distal end portion (41) and the rear end portion (42). A rear end (45) of the core wire (4) extends at least to a rear end (33a) of the gradient opening (33), so as to prevent the balloon catheter 1 from having a propensity of being easily bent.

## Description

The invention relates to a balloon catheter which is inserted into the somatic cavity (e.g., blood vessel and the like) to therapeutically dilate an occluded area of the blood vessel or to shut off the blood streams.

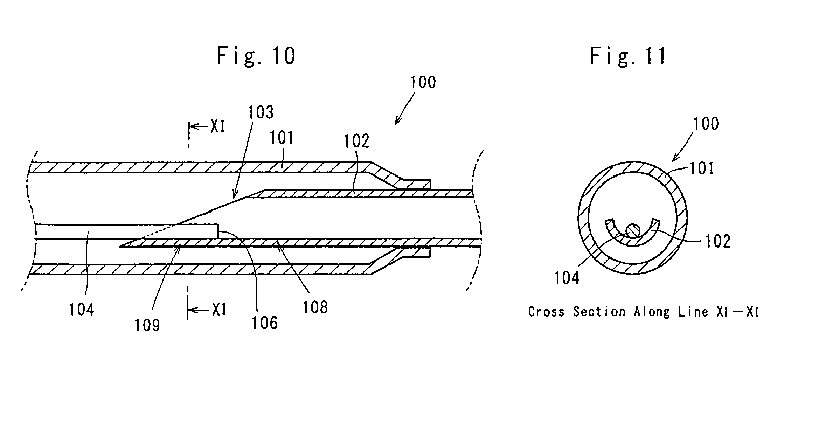

In a prior balloon catheter 100 which has a flexible shaft 101 as shown in Figs. 10 and 11, a distal end portion of the shaft 101 has a balloon (not shown). To a rear end side of the shaft 101, connected is a hypotube 102 of high rigidity as disclosed by Japanese Laid-open Patent Application No.2003-164528.

In order to alleviate an abrupt change of rigidity occurred between the hypotube 102 and the shaft 101, a distal end portion 103 of the hypotube 102 is severed aslant against its axial direction, so as to gradually decrease the rigidity as approaching forward. A core wire 104 is provided, a distal end portion of which is inserted into the shaft 101. A rear end side of the core wire 104 is fixedly placed at the distal end portion 103 of the hypotube 102.

In the above balloon catheter, it is necessary to diametrically reduce the core wire 104 so as to insure a sufficient cross sectional area for a fluid passage between the rear end side of the core wire 104 and the distal end portion 103 of the hypotube 102 when injecting a liquid flowing through the fluid passage to therapeutically dilate the balloon.

For this reason, it is still difficult to enhance the rigidity of the portion of the balloon catheter 100 in which only the core wire 104 resides under the absence of the hypotube 102. This yet retains the sharp change of the rigidity at the boundary between the portion of the balloon catheter 100 in which the hypotube 102 resides and the portion of the balloon catheter 100 in which the hypotube 102 is absent.

In this situation, upon inserting the balloon catheter 100 into the sinuous or curved blood vessel, the balloon catheter 100 has a propensity of being easily bent and broken at the boundary between the portion under the presence of the hypotube 102 and the portion under the absence of the hypotube 102.

The structure is such that it becomes difficult to make the liquid flow in and out through the fluid passage, so as to eventually delay the dilatation and deflation of the balloon more as the core wire 104 is placed to move farther into the rear side of the hypotube 102.

In order to avoid the delay of the dilatation and deflation of the balloon due to the restricted liquid flow, it is necessary to place a rear end 106 of the core wire 104 at a half way of the slantingly severed end 103 of the hypotube 102.

In this case, there arises a portion 108 in which the core wire 104 does not reside at the slantingly severed end 103. Due to a greater rigidity difference appeared between the portion 108 and a portion 109 in which the core wire 104 resides at the slantingly severed end 103, the balloon catheter 100 still has the propensity of being easily bent.

Therefore, it is an object of the invention to overcome the above drawbacks so as to provide a balloon catheter which has a hypotube at a rear side of a flexible hollow shaft, and is capable to avoid the balloon catheter from having a propensity of being easily bent.

### SUMMARY OF THE INVENTION

According to the present invention, there is provided a balloon catheter which has a flexible hollow shaft, a distal end of which has a balloon. A hypotube is provided, a distal end portion of which is inserted into a rear end side of the shaft. The hypotube has a lumen which acts as a fluid passage, through which a liquid flows in the balloon and flows out of the balloon alternately.

A core wire is provided, a distal end portion of which is inserted into a distal end side of the shaft. The core wire has a rear end positioned on a rear end side of the distal end portion to be fixedly inserted into the distal end portion of the hypotube. The distal end portion of the hypotube has a gradient opening, an open surface end of which inclines against an axial direction of the hypotube.

A rear end portion of the core wire has a cross sectional area smaller than a cross sectional area of a rear end side of the distal end portion. A rear end of the core wire positionally extends at least to a rear end of the gradient opening.

With the cross sectional area of the rear end portion of the core wire determined to be smaller than the cross sectional area of the rear end side of the distal end portion, it is possible to insure a sufficient fluid passage area which permits the liquid to flow in and out through the distal end portion of the hypotube. This enables a user to achieve a smooth inflow and outflow of the liquid through the distal end portion of the hypotube, while at the same time, making it possible to place a diametrically increased core wire inside the shaft.

As a result, it becomes possible to enhance the rigidity of the portion of the balloon catheter in which the core wire resides under the absence of the hypotube. This alleviates an abrupt change of the rigidity at the boundary between the portion of the balloon catheter in which the hypotube resides and the portion of the balloon catheter in which the hypotube does not reside, thus avoiding the balloon catheter from having a propensity of being easily bent.

With the rear end of the core wire positionally extended at least to the rear end of the gradient opening, there arises no place in which the core wire does not reside within the gradient opening located at the distal end portion of the hypotube. Even from this point of view, it is possible to avoid the balloon catheter from having the propensity of being easily bent.

According to other aspect of the invention, the rear end portion of the core wire is tapered in registration with an open end side of the hypotube, so as to progressively decrease a diametrical width as approaching a rear end side of the hypotube. This enables the user to insure a sufficient fluid passage area at the side of the gradient opening so as to attain a smooth inflow and outflow of the liquid through the distal end portion of the hypotube.

According to other aspect of the invention, the rear end portion of the core wire is shaped into an angular configuration.

According to other aspect of the invention, the distal end portion of the hypotube has the gradient opening, the open surface end of which extends at least to a diametrical central portion of the distal end portion, and further having a planar opening formed by axially cutting the distal end portion forward at least from the diametrical central portion of the distal end portion.

According to other aspect of the invention, a helical hollow spring body is provided to surroundingly cover the outer surface of the gradient opening and the planar opening.

The helical hollow spring body can compensate a reduced strength of the hypotube caused by providing the gradient opening and the planar opening. The helical hollow spring body prevents an open end of the planar opening from distortedly spreading in the diametrical direction when the balloon catheter is bent upon inserting the balloon catheter into the sinuous or curved blood vessel. This alleviates a bend-oriented inclination of the balloon catheter, and restrains the balloon catheter from having the propensity of being easily bent.

According to other aspect of the invention, the helical hollow spring body has a single wire element or a plurality of wire elements each tabular in cross section. This makes it possible to reduce a breadth of the wire element in the diametrical direction of the helical hollow spring body, so as to decrease a thickness of a wall of the helical hollow spring body.

A non-limiting example of the present invention is illustrated in the accompanying drawings in which:
Fig. 1 is a longitudinal cross sectional view of a balloon catheter according to a first embodiment of the invention;
Fig. 2 is a longitudinal cross sectional view of a main portion of the balloon catheter;
Fig. 3 is a latitudinal cross sectional view taken along the line III-III of Fig. 2;
Fig. 4 is a plan view of a core wire according to a second embodiment of the invention;
Fig. 5 is a side elevational view of the core wire according to the second embodiment of the invention;
Fig. 6 is a plan view of the core wire according to a third embodiment of the invention;
Fig. 7 is a side elevational view of the core wire according to the third embodiment of the invention;
Fig. 8 is a plan view of the core wire according to a fourth embodiment of the invention;
Fig. 9 is a side elevational view of the core wire according to the fourth embodiment of the invention;
Fig. 10 is a longitudinal cross sectional view of a prior balloon catheter; and
Fig. 11 is a latitudinal cross sectional view taken along the line XI-XI of Fig. 10.

In the following description of the depicted embodiments, the same reference numerals are used for features of the same type.

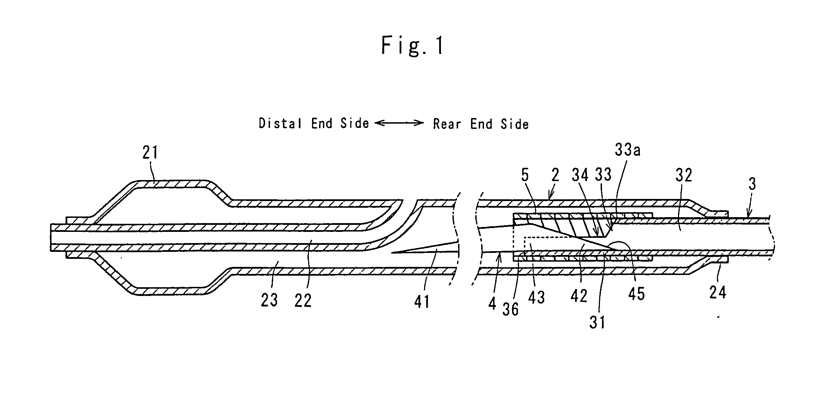

Referring to Figs. 1 through 3 which show a balloon catheter 1 according to a first embodiment of the invention. The balloon catheter 1 has a flexible hollow shaft 2, a hypotube 3 and a core wire 4 as shown in Fig. 1.

The hypotube 3 is inserted into a rear end side of the shaft 2. A distal end side of the core wire 4 is inserted into the shaft 2, and a rear end side of the core wire 4 is fixedly inserted into the hypotube 3.

The shaft 2 is shaped into a tubular configuration from such materials as, for example, polyamide, polyamide-based elastomer, polyolefine, polyester or polyester-based elastomer. A distal end of the shaft 2 has a balloon 21. Within the shaft 2, a guiding lumen 22 and a fluid lumen 23 are provided. The guiding lumen 22 is to introduce a guide wire (not shown) in the shaft 2, and the fluid lumen 23 is to act as a fluid passage, through which a liquid flows in and out to alternately dilate and deflate the balloon 21.

The hypotube 3 is shaped into a tubular configuration from such materials of high rigidity as, for example, stainless steel or superelastic alloy. A distal end portion 31 of the hypotube 3 is inserted into the rear end side of the shaft 2 with the hypotube 3 liquid-tightly fixed to an inner surface of a rear end of the shaft 2. An inner space of the hypotube 3 serves as a lumen 32 which is in communication with the fluid lumen 23 to act as a fluid path, through which the liquid flows in and out of the balloon 21 alternately.

A distal end portion 31 of the hypotube 3 has a gradient opening 33, an open surface end of which inclines distally to angle down against an axial direction of the hypotube 3. An open surface end of the gradient opening 33 extends at least to a diametrical central portion of the distal end portion 31 from an outer periphery of the distal end portion 31.

The distal end portion 31 further has a planar opening 34 formed by axially cutting the distal end portion 31 forward at least from the diametrical central portion of the hypotube 3 to an open end of the distal end portion 31.

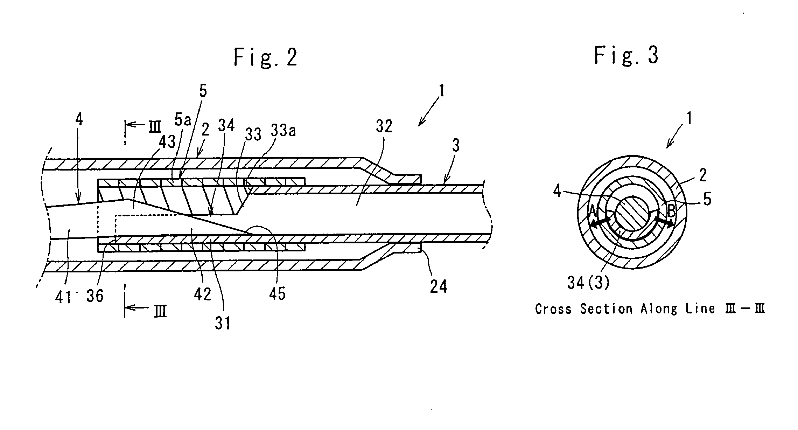

The planar opening 34 leaves a half cylindrical portion on the distal end portion 31 of the hypotube 3 as shown in Figs. 2 and 3.

The core wire 4 is made from such materials as, for example, stainless steel or superelastic alloy, and having a distal end portion 41 and a rear end portion 42. The distal end portion 41 is tapered to be diametrically reduced as approaching forward. The rear end portion 42 extends from a rear end side of the distal end portion 41, and is fixedly inserted into the distal end portion 31 of the hypotube 3.

In the embodiment of the invention, an entire portion of the distal end portion 41 is tapered, however, an equi-diametrical portion may be partly formed inclusively in the distal end portion 41. The equi-diametrical portion means that its outer diameter remains unchanged along the lengthwise direction. The rear end portion 42 of the core wire 4 is tapered at an open end side of the hypotube 3, so as to progressively decrease a diametrical width as approaching a rear end side of the hypotube 3.

With the result that the rear end portion 42 is tapered, the rear end portion 42 of the core wire 4 has a cross sectional area smaller than a cross sectional area of a boundary 43 (a rear end of the distal end portion 41) bet ween the distal end portion 41 and the rear end portion 42. The rear end portion 42 progressively decreases its cross sectional area as approaching rearward.

The core wire 4 has a rear end 45 positionally extended to a rear end 33a of the gradient opening 33. The rear end 45 may extends rearward beyond the rear end 33a of the gradient opening 33.

A boundary 43 is designated between the distal end portion 41 and the rear end portion 42 of the core wire 4. It is necessary to place the boundary 43 at a distal end side of the gradient opening 33 to permit a smooth liquid flow through the boundary 43.

In this instance, the boundary 43 is situated in the proximity of a front end 36 of the hypotube 3. More precisely, the boundary 43 is located slightly close to a rear side of the front end 36 of the hypotube 3.

The rear end portion 42 of the core wire 4 is secured to an inner wall of a distal end portion 31 of the hypotube 3 by means of a heat welding procedure (e.g., laser welding, soldering, brazing or the like). The balloon catheter 1 has a helical hollow spring body 5 provided to surround outer surfaces of the gradient opening 33 and the planar opening 34. The helical hollow spring body 5 is formed by helically winding a plurality of wire elements 5a, and located at the outer surface of the hypotube 3 to cover the gradient opening 33 and the planar opening 34. The wire elements 5a are tabular (rectangular) in cross section.

By way of illustration, an inner diameter of a rear side of the shaft 2 measures 0.78 mm with an inner diameter and outer diameter of the hypotube 3 designated in turn as 0.48 mm and 0.62 mm. An outer diameter of the core wire 4 measures 0.45 mm at the boundary 43 between the distal end portion 41 and the rear end portion 42 of the core wire 4. Depending on a length of the balloon 21 , the shaft 2 measures from 346 mm to 358 mm in length.

The planar opening 34 measures 8 mm in length, and the gradient opening 33 measures 2 mm in length each along the axial direction of the shaft 2. The distal end portion 41 and the rear end portion 42 of the core wire 4 respectively measures 100 mm and 7 mm in length.

With the structure thus far described, the rear end portion 42 of the core wire 4 is tapered at the portion in which the rear end portion 42 faces the open-end portion of the hypotube 3. Namely, the rear end portion 42 is tapered in registration with the open end side of the hypotube 3 so as to be diametrically reduced progressively as approaching rearward.

With the result that the rear end portion 42 is tapered, the cross sectional area of the rear end portion 42 of the core wire 4 is determined to be smaller than the cross sectional area of the boundary 43, so as to insure a sufficient fluid passage area which permits the liquid to flow in and out through the distal end portion 31 of the hypotube 3.

With the above structure, it is possible to achieve a smooth inflow and outflow of the liquid through the distal end portion 31 of the hypotube 3, while at the same time, permitting to set a diametrically increased core wire inside the shaft 2.

This enables the user to place the diametrically increased core wire inside the shaft, so as to enhance the rigidity of the portion of the balloon catheter in which the core wire 4 resides. This alleviates the abrupt change of the rigidity at the boundary 43 between the portion of the balloon catheter 1 in which the hypotube 3 resides and the portion of the balloon catheter 1 in which the hypotube 3 does not reside, thus avoiding the balloon catheter 1 from having a propensity of being easily bent.

With the rear end 45 of the core wire 4 positionally extended to the rear end 33a of the gradient opening 33, there arises no place in which the core wire 4 does not reside within the gradient opening 33 located at the distal end portion 31 of the hypotube 3. Namely, the core wire 4 invariably resides inside the distal end portion 31 of the hypotube 3, so as to avoid the balloon catheter 1 from having the propensity of being easily bent.

With the helical hollow spring body 5 provided to surroundingly cover the gradient opening 33 and the planar opening 34, it is possible to compensate a reduced strength of the hypotube 3 caused by providing the gradient opening 33 and the planar opening 34.

The helical hollow spring body 5 prevents an open end of the planar opening 34 from distortedly spreading in the diametrical direction as designated by arrows A and B in Fig. 2 when the balloon catheter 1 is bent upon inserting the balloon catheter 1 into the sinuous or curved blood vessel. This alleviates a bend-oriented inclination of the balloon catheter 1, and restrains the balloon catheter 1 from having the propensity of being easily bent.

With the wire elements 5a of the helical hollow spring body 5 being tabular in cross section, it is possible to reduce a breadth of the wire elements 5a in the diametrical direction of the helical hollow spring body 5, so as to decrease a thickness of a wall of the helical hollow spring body 5. Because of the thickness-reduced wall of the helical hollow spring body 5, it is possible to set the helical hollow spring body 5 even into a narrow interstice between an inner wall of the shaft 2 and an outer wall of the hypotube 3.

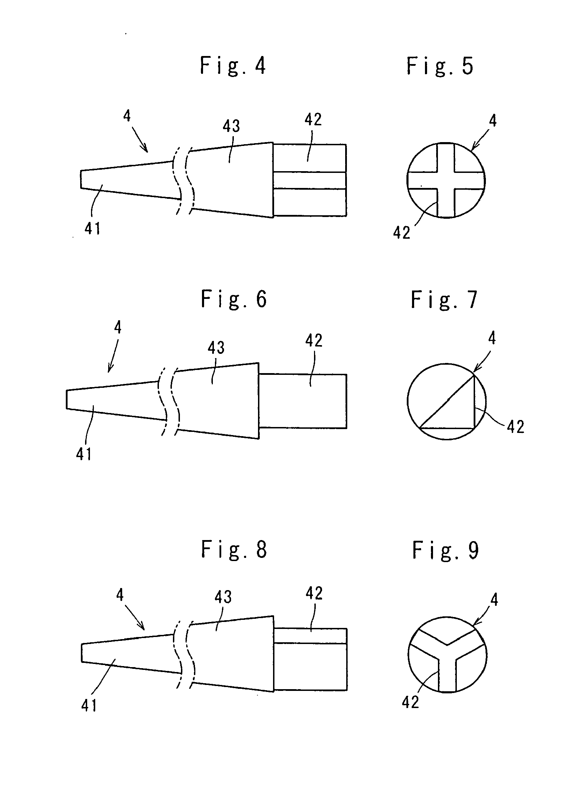

Figs. 4 and 5 show a second embodiment of the invention in which the rear end portion 42 of the core wire 4 is angular in shape, and having a cruciform cross section. The rear end portion 42 of the core wire 4 has a cross sectional area smaller than a cross sectional area of the boundary 43 between the distal end portion 41 and the rear end portion 42 of the core wire 4.

Figs. 6 and 7 show a third embodiment of the invention in which the rear end portion 42 of the core wire 4 is angular in shape, and having a cross section of a right-angled triangle. The rear end portion 42 of the core wire 4 has a cross sectional area smaller than a cross sectional area of the boundary 43 between the distal end portion 41 and the rear end portion 42 of the core wire 4.

Figs. 8 and 9 show a fourth embodiment of the invention in which the rear end portion 42 of the core wire 4 is angular in shape, and having a Y-shaped cross section. The rear end portion 42 of the core wire 4 has a cross sectional area smaller than a cross sectional area of the boundary 43 between the distal end portion 41 and the rear end portion 42 of the core wire 4.

According to the second, third and fourth embodiments of the invention, it is possible to insure a sufficient space as the fluid passage between the core wire 4 and an inner wall of the hypotube 3.

### MODIFICATION FORMS

(a) Upon forming the gradient opening 33, the distal end portion 31 of the hypotube 3 may be cut slantwisely to extend to a distal open end of the hypotube 3 without providing the planar opening 34.
(b) Instead of providing the plurality of the wire elements 5a to form the helical hollow spring body 5, only one single wire element may be provided to form the helical hollow spring body 5.
   It is, however, preferable to use the plurality of the wire elements 5a because they reinforce the physical strength of the helical hollow spring body 5.
(c) Upon providing the shaft 2 in which the core wire 4, the hypotube 3 and the helical hollow spring body 5 are provided, a molten synthetic resin (not shown) may be poured into helical interstices between the wire elements 5a of the helical hollow spring body 5, so as to form the helical hollow spring body 5, the interstices of which are filled by the synthetic resin. This makes the balloon catheter 1 preferably resist against an expansile force because the helical hollow spring body 5 is strengthened at its stretch-oriented resistance.

## Claims

1. A balloon catheter (1) which has a flexible hollow shaft (2), a distal end of which has a balloon (21);
**characterized in that**,
a hypotube (3) is provided, a distal end portion (31) of which is inserted into a rear end side of said shaft (2), said hypotube (3) having a lumen (32) which acts as a fluid passage, through which a liquid flows in said balloon (21) and flows out of said balloon (21) alternately;
a core wire (4) is provided, a distal end portion (41) of which is inserted into a distal end side of said shaft (2), said core wire (4) having a rear end portion (4 2) positioned on a rear end side of said distal end portion (41) to be fixedly inserted into said distal end portion (31) of said hypotube (3);
said distal end portion (31) of said hypotube (3) has a gradient opening (33), an open surface end of which inclines against an axial direction thereof;
said rear end portion (42) of said core wire (4) has a cross sectional area smaller then a cross sectional area of a rear end side of said distal end portion (41); and
a rear end (45) of said core wire (4) positionally extends at least to a rear end (33a) of said gradient opening (33).

2. The balloon catheter (1) according to claim 1, wherein said rear end portion (42) of said core wire (4) is tapered in registration with an open end side of said hypotube (3), so that a diametrical width progressively decreases as approaching a rear end side of said hypotube (3).

3. The balloon catheter (1) according to claim 1, wherein said rear end portion (42) of said core wire (4) is shaped into an angular configuration.

4. The balloon catheter (1) according to any of claims 1-3, wherein said distal end portion (31) of said hypotube (3) comprises said gradient opening (33), the open surface end of which extends at least to a diametrical central portion of said distal end portion (31), and a planar opening (34) formed by axially cutting said distal end portion (31) forward at least from said diametrical central portion thereof.

5. The balloon catheter (1) according to claim 4, wherein a helical hollow spring body (5) is provided to surroundingly cover said outer surface of said gradient opening (33) and said planar opening (34).

6. The balloon catheter (1) according to claim 5, wherein said helical hollow spring body (5) has a single wire element or a plurality of wire elements (5a) each tabular in cross section.
